# EUROPEAN PATENT APPLICATION

(11) **EP 4 317 974 A1**
(43) Date of publication of application: **07.02.2024**
(21) Application number: 22775171.6
(22) Date of filing: 10.03.2022
(51) Int. Cl.: G01N 33/543

(54) **TEST CARTRIDGE**

(30) Priority: 26.03.2021 JP 2021054326
(71) Applicant: FUJIFILM Corporation, Tokyo 106-8620 (JP)
(72) Inventor: SETO, Yoshihiro, Ashigarakami-gun, Kanagawa 258-8538 (JP); ISHII, Hiroyasu, Ashigarakami-gun, Kanagawa 258-8538 (JP)
(74) Representative: Hughes, Andrea Michelle
(86) International application number: PCT/JP2022/010716
(87) International publication number: WO 2022/202379

(57) **Abstract**

An assay cartridge is an assay cartridge used for immunochromatographic assay, including a carrier to which a sample and a reagent are supplied, an enclosing body in which a reagent to be supplied to the carrier is enclosed, and which has a sealing portion that opens earlier than other portions in a case where an internal pressure is increased due to an application of a pressing force, and a case accommodating the carrier and the enclosing body and having a pressing operation part for applying the pressing force to the enclosing body.

## Description

### BACKGROUND OF THE INVENTION

### 1. Field of the Invention

The technique of the present disclosure relates to an assay cartridge.

### 2. Description of the Related Art

WO2017/104143A describes an immunochromatographic kit for performing an assay of whether a sample is positive or negative, that is, whether or not the sample contains a test substance, using an immunochromatographic method. This immunochromatographic kit is called an assay cartridge or the like. The immunochromatographic kit described in WO2017/104143A includes an immunochromatographic carrier (hereinafter, referred to as a carrier) to which a sample and a reagent such as an amplifying liquid are supplied, and a case in which the carrier is accommodated. Furthermore, a pod in which a reagent is sealed is accommodated in the case. The pod has a container having an opening on one surface, and the opening portion of the container is covered with a sheet member to seal the container. The pod is disposed in a posture in which the sheet member and the carrier face each other, and a protrusion is provided between the sheet member and the carrier.

A pressing button is provided in the case, and in a case where the pressing button is operated, a pressing force for pushing the container toward the carrier side is applied to the container. In a case where the container is pressed toward the carrier side, the sheet member is pushed to the protrusion to break a part of the sheet member due to this pushing. The reagent is discharged to the outside of the pod due to the breakage of the sheet member.

### SUMMARY OF THE INVENTION

An assay cartridge such as the immunochromatographic kit described in WO2017/104143A may be used by being attached to an assay apparatus, or may be used alone without using the assay apparatus. The pressing button provided in the case may be operated by an internal mechanism of the assay apparatus or may be operated by a person.

The pressing force, the pressing speed, and the position where the pressing force is applied in a case where the pressing button is operated are different between the case where the pressing button is operated by the assay apparatus and the case where the pressing button is operated by a person. In addition, even in a case of being operated by a person, there are individual differences in the pressing force and the like. In this way, in a case where the pressing force and the like are different, the position, the size, the shape, and the like of the broken portion of the sheet member are changed, and thus there is a problem that the discharge direction of the reagent is not stable. In a case where the discharge direction of the reagent is not stable, the amount of the reagent scattered in a direction different from that to the carrier increases, and the amount of wasted reagent that is not supplied to the carrier increases. Since many expensive reagents are used, there has been a demand to reduce the waste of reagents as much as possible.

In consideration of the above-described fact, the technique according to the present disclosure provides an assay cartridge in which a wasted reagents are less likely to be generated.

The assay cartridge of the present disclosure is an assay cartridge used for immunochromatographic assay, including a carrier to which a sample and a reagent are supplied, an enclosing body in which the reagent to be supplied to the carrier is enclosed, and which has a sealing portion that opens earlier than other portions in a case where an internal pressure is increased due to an application of a pressing force, and a case accommodating the carrier and the enclosing body and having a pressing operation part for applying the pressing force to the enclosing body.

The assay cartridge of the present disclosure preferably includes a pressing part that applies the pressing force by being pressed against the enclosing body in a case where the pressing operation part is operated, and in which an abutting surface abutting on the enclosing body is a flat surface.

In the assay cartridge of the present disclosure, it is preferable that in the case, an accommodating part that accommodates the enclosing body and in which a supply port for supplying the reagent discharged from the enclosing body to the carrier is formed, is provided, and in the accommodating part, the sealing portion is disposed at the supply port.

In the assay cartridge of the present disclosure, a position of the sealing portion disposed at the supply port is preferably fixed.

In the assay cartridge of the present disclosure, it is preferable that in the case, an accommodating part that accommodates the enclosing body and in which a supply port for supplying the reagent discharged from the enclosing body to the carrier is formed, is provided, and in the accommodating part, the sealing portion is connected to the supply port through a connecting portion.

In the assay cartridge of the present disclosure, it is preferable that the connecting portion is a portion of the enclosing body and is a portion extended from the sealing portion toward the supply port.

In the assay cartridge of the present disclosure, the connecting portion is preferably a tubular body separated from the enclosing body.

In the assay cartridge of the present disclosure, it is preferable that in a case where the enclosing body includes a plurality of sealing portions, one of the plurality of the sealing portions is a weak sealing portion having a weaker sealing force than the other sealing portions, and the weak sealing portion is disposed at the supply port or is connected to the supply port through the connecting portion.

In the assay cartridge of the present disclosure, the sealing portion is preferably welded.

In the assay cartridge of the present disclosure, the sealing portion is preferably heat-welded.

In the assay cartridge of the present disclosure, sealing forces of the weak sealing portion and the other sealing portions are preferably adjusted by controlling an applied thermal energy.

In the assay cartridge of the present disclosure, the weak sealing portion preferably has a smaller welding area than the other sealing portions.

In the assay cartridge of the present disclosure, it is preferable that the enclosing body is formed of one sheet rolled into a cylindrical shape, the enclosing body includes two sealing portions that is formed along a direction intersecting a cylinder axis direction of the sheet and that is used for defining an enclosing body in which the reagent is to be enclosed, and one of the two sealing portions is the weak sealing portion.

According to the assay cartridge of the present disclosure, wasted reagents are less likely to be generated as compared with the prior art.

### BRIEF DESCRIPTION OF THE DRAWINGS

Fig. 1 is a perspective view of an assay cartridge according to the present disclosure.
Fig. 2 is an exploded perspective view of the assay cartridge according to the present disclosure.
Fig. 3A is a partially broken side view showing a state in which a first pressing operation part of the assay cartridge according to the present disclosure is pushed in, and Fig. 3B is a partially broken side view showing a state in which the first pressing operation part and a second pressing operation part are pushed in.
Fig. 4 is a side view showing a positional relationship between an assay strip, a multifunctional member, a first reagent holding part, and a second reagent holding part, in the assay cartridge according to the present disclosure.
Fig. 5 is an explanatory diagram of an immunochromatographic method.
Fig. 6A is a cross-sectional view showing a first displacement part, a second displacement part, and a second reagent holding part of the assay cartridge according to the present disclosure, and Fig. 6B is a cross-sectional view showing a state in which the first displacement part and the second displacement part are displaced and the second reagent is discharged from the second reagent holding part.

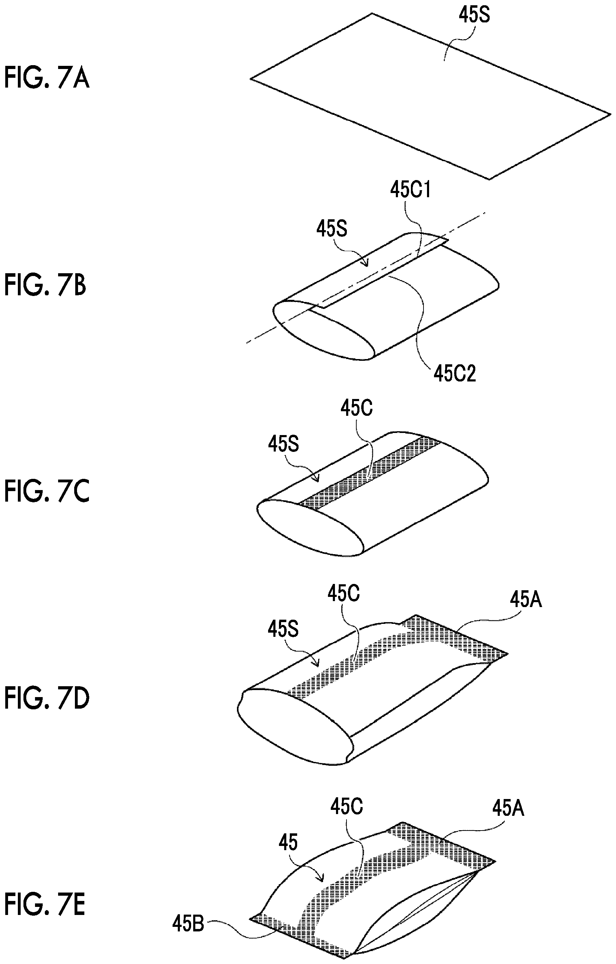

Fig. 7A is a perspective view showing a sheet member for manufacturing the second reagent holding part applied to the assay cartridge according to the present disclosure, Fig. 7B is a perspective view showing a state in which the sheet member is rolled into a cylindrical shape, Fig. 7C is a perspective view showing a state in which an end portion along the cylinder axis direction is welded, Fig. 7D is a perspective view showing a state in which one end portion in the cylinder axis direction is welded, and Fig. 7E is a perspective view showing a state in which the other end portion in the cylinder axis direction is welded.
Fig. 8 is a plan view showing a multifunctional member of the assay cartridge according to the present disclosure.
Fig. 9 is a cross-sectional view showing a state in which a sealing portion of the second reagent holding part of the assay cartridge according to the present disclosure is fixed to the second accommodating part.
Fig. 10A is a plan view showing an example in which a connecting part of a tubular body is formed in the second reagent holding part of the assay cartridge according to the present disclosure, and Fig. 10B is a cross-sectional view.
Fig. 11A is a plan view showing a state in which a part of the second reagent holding part of the assay cartridge according to the present disclosure is formed as a connecting portion and the connecting portion is inserted into a supply port of the second accommodating part, and Fig. 11B is a cross-sectional view.
Fig. 12A is a cross-sectional view showing a modified example in which a position of the first displacement part of the assay cartridge according to the present disclosure is changed, and Fig. 12B is a cross-sectional view showing a state in which the second reagent holding part is pressed.
Fig. 13A is a cross-sectional view showing a modified example in which one end portion of the second displacement part of the assay cartridge according to the present disclosure is defined as a fixed end, and Fig. 13B is a cross-sectional view showing a state in which the second reagent holding part is pressed.
Fig. 14A is a cross-sectional view showing a modified example in which a regulating part is provided in the second displacement part of the assay cartridge according to the present disclosure, Fig. 14B is a cross-sectional view showing a state in which a second reagent holding part is pressed, and Fig. 14C is a cross-sectional view taken along the line C-C in Fig. 14A.
Fig. 15A is a cross-sectional view showing a modified example in which the first displacement part of the assay cartridge according to the present disclosure is formed by a plurality of protrusions, and Fig. 15B is a cross-sectional view showing a state in which the second reagent holding part is pressed.

### DESCRIPTION OF THE PREFERRED EMBODIMENTS

Hereinafter, an assay cartridge according to an embodiment of the present invention will be described with reference to the drawings. The constituent elements indicated by the same reference numerals in the drawings mean the same constituent elements. However, unless otherwise specified in the specification, each component is not limited to one, and a plurality of each component may be present.

In addition, description of overlapping configurations and reference numerals in the respective drawings may be omitted. The present invention is not limited to the following embodiments, and can be implemented with appropriate modifications, such as omitting a configuration or replacing a configuration with a different configuration within the scope of the object of the present invention.

The directions indicated by the arrows X and Y, which are appropriately shown in the respective figures, are directions along the horizontal plane and are orthogonal to each other. In addition, the direction indicated by the arrow Z is a direction along the perpendicular direction (vertical direction). The directions indicated by the arrows X, Y, and Z in respective figures coincide with each other.

### <Overview of assay cartridge>

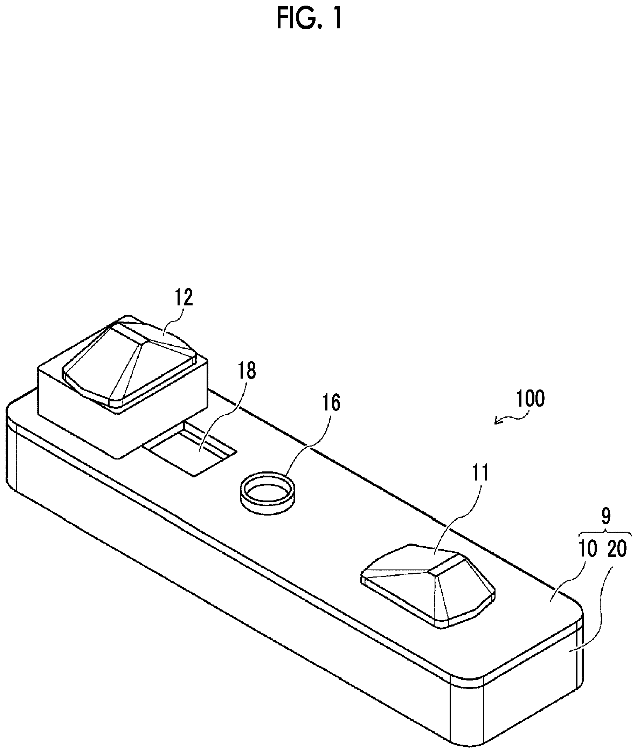

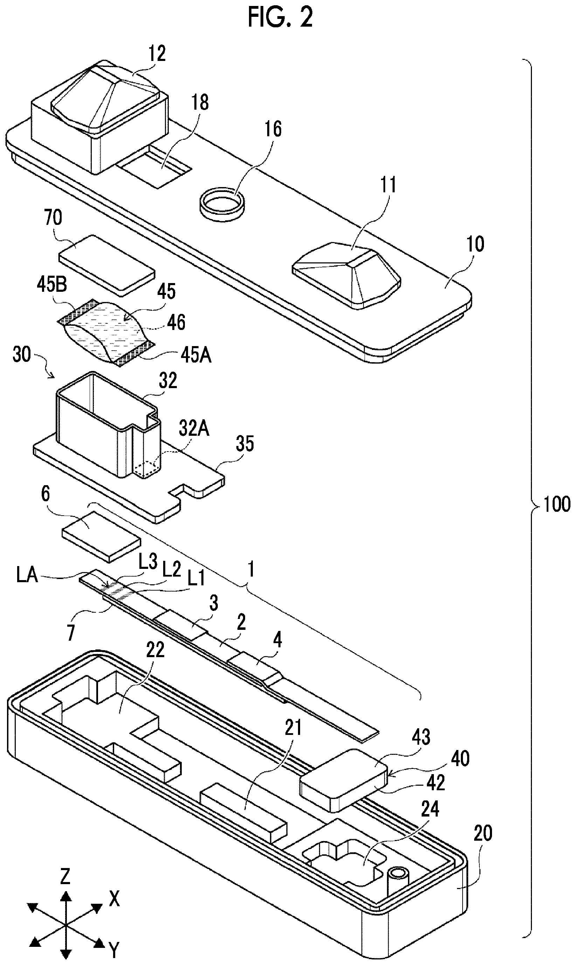

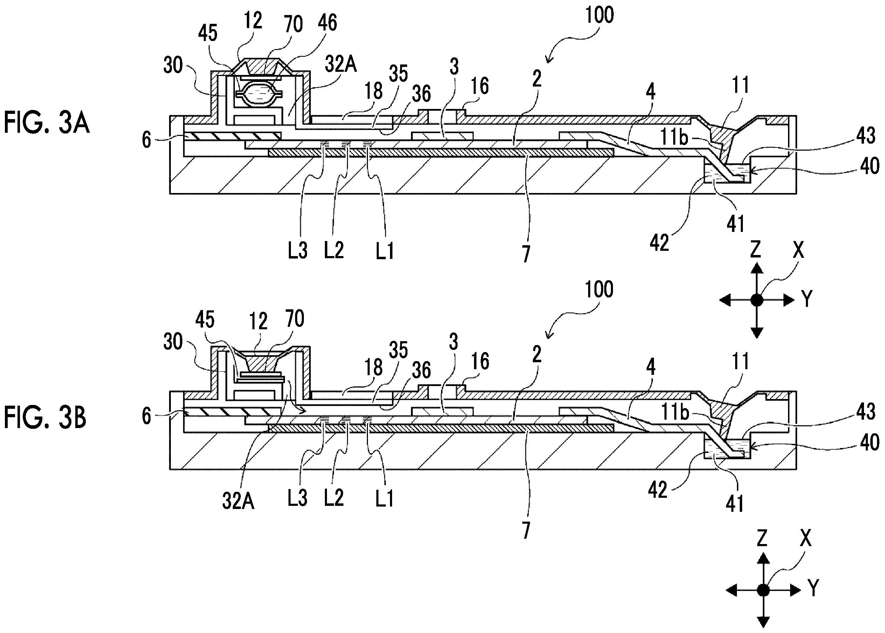

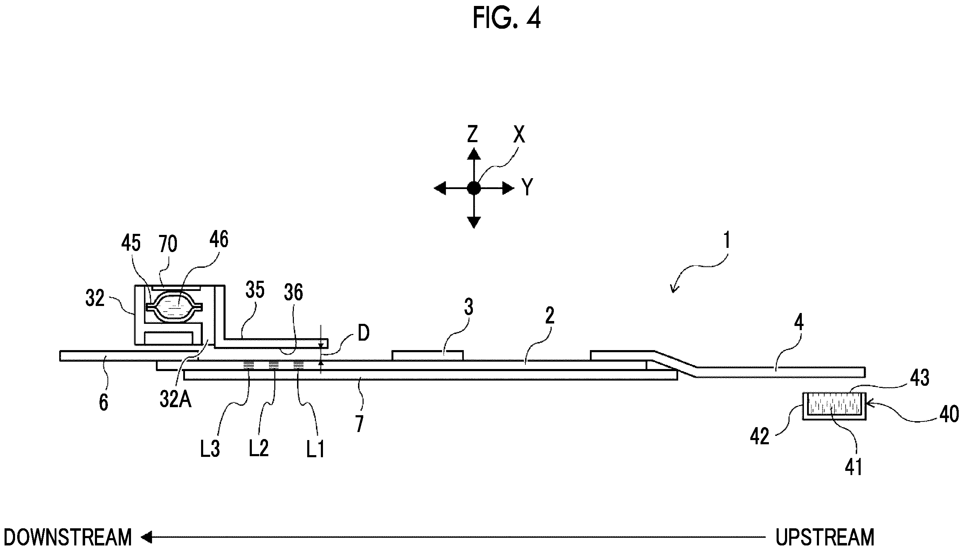

Fig. 1 is an external view of a cartridge 100 which is an assay cartridge according to one embodiment, and Fig. 2 is an exploded perspective view of the cartridge 100. Fig. 3 is a diagram showing a state in which the first pressing operation part 11 and the second pressing operation part 12 provided in the cartridge 100 are operated. Fig. 4 is a diagram showing the main accommodated components in the cartridge 100.

The cartridge 100 is a single-use type that is used one by one in each sample of assay target. As shown in Fig. 2, an assay strip 1 including an immunochromatographic carrier 2 (hereinafter, referred to as a carrier 2) is provided in the cartridge 100. An assay region L1 is provided in the carrier 2, and the color development state changes depending on whether or not the sample contains a test substance, that is, whether the sample is positive or negative.

The "change in color development state" includes any of an aspect in which a first color different from the color of the carrier 2 changes to another second color (that is, a color change), an aspect in which the color of the carrier 2 changes to another color by developing a color different from that of the carrier 2 (that is, color development), and an aspect in which the density of the color changes (that is, a change in density).

The sample is simply required to be a specimen that may contain a test substance, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, or a liquid specimen containing animals and plants themselves or a dried body thereof. Examples of the test substance include an antigen, an antibody, a protein, and a low-molecular-weight compound.

The cartridge 100 has a configuration that allows a user to visually confirm whether the sample is positive or negative. Such a cartridge 100 is also referred to as an immunochromatographic assay tool, an immunochromatographic assay kit, or the like.

As shown in Fig. 1 and Fig. 2, as an example, the cartridge 100 includes a case 9 constituted of a case body 20 and a cover member 10. The case 9 is formed of, for example, a resin material. An opening is formed in an upper part of the case body 20, and in addition to the assay strip 1, a first reagent holding part 40, a second reagent holding part 45, and the like are accommodated therein. The cover member 10 covers the opening of the case body 20 by being attached to the opening part of the case body 20. The case 9 has an elongated shape as a whole in accordance with the elongated shape of the assay strip 1.

In the present example, a dropping port 16, an observation window 18, a first pressing operation part 11, and a second pressing operation part 12 are provided on an upper part of the case 9 constituted of the cover member 10. Each of these parts is integrally molded with the cover member 10 as an example. The dropping port 16 is an opening for adding dropwise a sample into the inside of the case 9. A boss is vertically provided on the edge of the dropping port 16 toward the upper part.

### (Observation window)

An observation window 18 is an opening portion for observing the assay region L1 from the outside, in the present example, the size of the observation window 18 is a size such that, in addition to the assay region L1, the control region L2 and the color development region L3, which will be described later, can also be observed. In the carrier 2, a region that includes the assay region L1, the control region L2, the color development region L3, and the peripheral region thereof and can be observed from the observation window 18 is referred to as an observation region LA. The user can confirm the observation region LA through the observation window 18. The user can confirm whether the sample is positive or negative by observing the color development state of the assay region L1 in the observation region LA.

### (First pressing operation part, second pressing operation part)

As shown in Fig. 2, Fig. 3A, and Fig. 3B, the first pressing operation part 11 is an operating part operated to supply the first reagent 41 in the first reagent holding part 40 to the carrier 2. The second pressing operation part 12 is an operating part operated to supply the second reagent 46 in the second reagent holding part 45 to the carrier 2. As will be described later, the first reagent 41 and the second reagent 46 are amplifying liquids for amplifying the color development in the assay region L1 in a case where the sample is positive.

As shown in Fig. 3A, in a case where a pressing force is applied from the outside as an external force to the first pressing operation part 11 by a pressing operation by a user or the like, the first pressing operation part 11 is deformed. As shown in Fig. 2, as an example, the first pressing operation part 11 has a quadrangular pyramid shape, and in a case where a pressing force is applied from above to a region including the apex of the quadrangular pyramid, as shown in Fig. 3A, the first pressing operation part 11 is deformed such that the apex of the quadrangular pyramid sinks into the inside of the case 9. In a case of where the first pressing operation part 11 is deformed in this manner, a pressing force is applied to the first reagent holding part 40 inside the case 9. In the first reagent holding part 40, deformation or the like due to a pressing force applied through the first pressing operation part 11 occurs. Due to this deformation or the like, the first reagent 41 held by the first reagent holding part 40 is supplied to the assay strip 1.

In addition, the first pressing operation part 11 is deformed by pressing and then the deformed state is maintained. Accordingly, after the first pressing operation part 11 is pressed, the supply of the first reagent 41 to the assay strip 1 is continued.

Similarly, as shown in Fig. 3B, in a case where a pressing force is applied from the outside as an external force to the second pressing operation part 12, the second pressing operation part 12 is deformed. As shown in Fig. 2, similarly to the first pressing operation part 11, the second pressing operation part 12 of the present example also has a quadrangular pyramid shape, and in a case where a pressing force is applied from above to a region including the apex of the quadrangular pyramid, as shown in Fig. 3B, the second pressing operation part 12 is deformed such that the apex of the quadrangular pyramid sinks into the inside of the case 9. In a case of where the second pressing operation part 12 is deformed in this manner, a pressing force is applied to the second reagent holding part 45 inside the case 9. In the second reagent holding part 45, deformation or the like due to a pressing force applied through the second pressing operation part 12 occurs. Due to this deformation or the like, the second reagent 46 held by the second reagent holding part 45 is supplied to the assay strip 1.

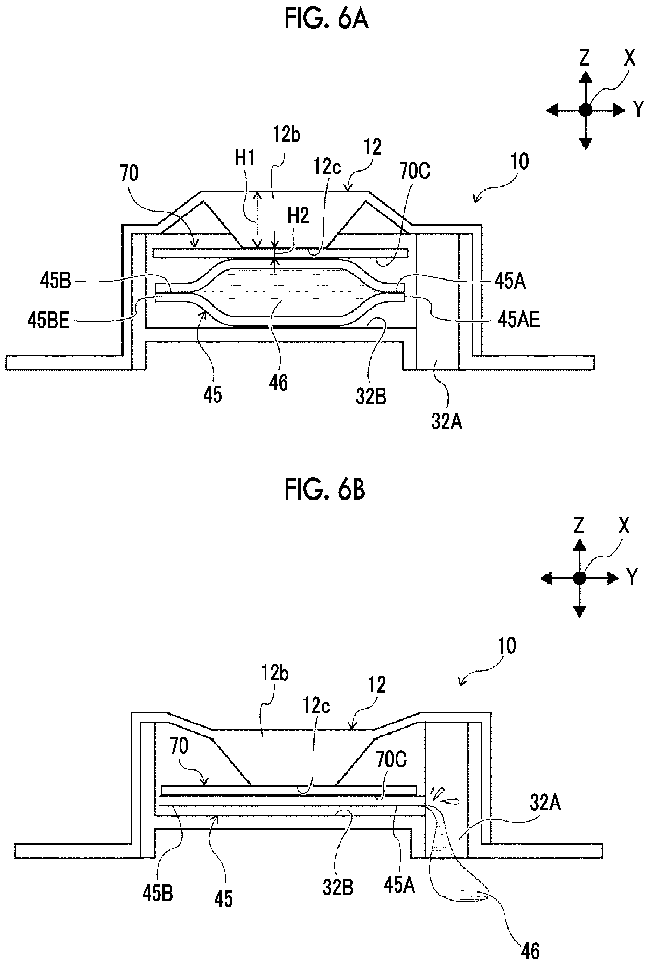

In this way, the second pressing operation part 12 is a pressing operation part operated to supply the second reagent 46 in the second reagent holding part 45 to the carrier 2 by applying the pressing force to the second reagent holding part 45. The second pressing operation part 12 of the present example is provided with a first displacement part 12b (see Fig. 6) that is displaced inside the case 9 in a case where the second pressing operation part 12 is operated. The first displacement part 12b is formed integrally with the second pressing operation part 12, and has a first pressing surface 12c (see Fig. 6) for pushing the second displacement part 70 to the second reagent holding part 45 in a case where the second pressing operation part 12 is operated. The second displacement part 70 is a plate material that can be displaced inside the case 9 including the cover member 10 in which the second pressing operation part 12 is formed, and inside the second accommodating part 32, which will be described later.

In addition, similarly to the first pressing operation part 11, preferably, the second pressing operation part 12 is deformed by pressing and then the deformed state is maintained, and the state in which the first displacement part 12b is displaced is maintained. It is because, in a case where the second pressing operation part 12 is pressed by the user, the second pressing operation part 12 in which the deformation is maintained even after the user releases the hand, is easier to continue the supply of the second reagent 46.

### (First reagent holding part)

As shown in Fig. 2, Fig. 3A, and Fig. 3B, the case body 20 accommodates the assay strip 1 including the carrier 2 along the longitudinal direction. As shown in Fig. 3A, Fig. 3B, and Fig. 4, in the case body 20, the first reagent holding part 40 is disposed on one end part side (upstream side shown in Fig. 4) in the longitudinal direction. In the case body 20, in a portion where the first reagent holding part 40 is disposed, the first accommodating part 24 that is a recess-shaped in accordance with the shape of the first reagent holding part 40 is formed. One end part of the assay strip 1 is disposed above the first reagent holding part 40 in a state of being accommodated in the first accommodating part 24.

As shown in Fig. 3A, Fig. 3B, and Fig. 4, the first reagent holding part 40 holds the first reagent 41. The first reagent holding part 40 is constituted of, for example, a container 42 formed of a resin material and having an opening on one surface, and a sheet member 43 that covers the opening of the container 42 and is breakable. The container 42 is filled with the first reagent 41, and the opening of the container 42 is sealed by the sheet member 43. The first reagent holding part 40 is disposed in the first accommodating part 24 in a posture in which the sheet member 43 faces upward.

The pressing force applied from the first pressing operation part 11 is transmitted to the sheet member 43 of the first reagent holding part 40 via the end part of the assay strip 1 to break the sheet member 43. The sheet member 43 is broken, and thus the first reagent 41 is supplied to the assay strip 1. In the first pressing operation part 11 of the present example, a protruding part 11b that abuts on the sheet member 43. The protruding part 11b has, for example, an elongated shape extending in the longitudinal direction in the width direction of the assay strip 1 and a pointed shape toward the sheet member 43, such that the sheet member 43 is easily broken.

### (Multifunctional member)

In addition, as shown in Fig. 2, Fig. 3A, and Fig. 3B, the cartridge 100 includes a multifunctional member 30 having a function of accommodating the second reagent holding part 45. The multifunctional member 30 is disposed on the other end part side (downstream side shown in Fig. 4) of the case body 20 and above the assay strip 1. The multifunctional member 30 is a member in which the second accommodating part 32 and the flow channel forming part 35 are integrally formed.

### (Multifunctional member - second accommodating part)

The second accommodating part 32 has a box shape having an opened upper surface. The second accommodating part 32 is provided inside the case 9 including the cover member 10. The second accommodating part 32 is an accommodating part accommodating the second reagent holding part 45, and is provided with a supply port 32A for supplying the second reagent 46 discharged from the second reagent holding part 45 to the carrier 2.

In the second accommodating part 32, a second displacement part 70 is disposed between the first displacement part 12b of the second pressing operation part 12 and the second reagent holding part 45. The second reagent holding part 45 is formed of a flexible material having flexibility as will be described later, and in a case where the second pressing operation part 12 is pressed, the second reagent holding part 45 is crushed via the second displacement part 70. In a case where the second reagent holding part 45 is crushed, the second reagent holding part 45 is opened and the second reagent 46 is discharged. The second reagent 46 is supplied to the carrier 2 from the supply port 32A.

### (Multifunctional member - flow channel forming part)

Furthermore, the flow channel forming part 35 is provided to be connected to the upstream side from the second accommodating part 32. The flow channel forming part 35 has a flat plate shape, is disposed at a position facing the assay region L1 or the like in the longitudinal direction of the assay strip 1, and is disposed with an interval from the assay strip 1. Then, between the flow channel forming part 35 and the assay strip 1, a flow channel for flowing the second reagent 46 flowed out from the second accommodating part 32 toward the assay region L1 or the like is formed. The flow channel forming part 35 is disposed between the observation window 18 and the assay region L1 or the like of the assay strip 1. Therefore, the flow channel forming part 35 is formed of a transparent member and thus the assay region L1 and the like can be observed through the observation window 18.

As will be described later (see Fig. 6 and below), the cartridge 100 of the present example is characterized in a structure of the second reagent holding part 45 and periphery thereof, but the characteristic of the second reagent holding part 45 and the like will be described after a basic configuration, a basic method for using, and the like of the cartridge 100 is described.

As shown in Fig. 4, a gap (a clearance) D corresponding to the flow channel for the second reagent 46 is formed between a back surface 36 of the flow channel forming part 35 of the multifunctional member 30 and the carrier 2 of the assay strip 1. The gap D is, for example, in the range of 0.01 mm to 1 mm. The second reagent 46 flows out from the supply port 32A as an opening at the bottom of the second accommodating part 32 toward the carrier 2, and the second reagent 46 that has flowed out flows through the flow channel formed by the gap D and reaches at least above the assay region L1. The second reagent 46 that has reached the assay region L1 infiltrates the assay region L1 from the flow channel.

An absorption pad 6, which will be described later, is disposed at an end part on the downstream side of the assay strip 1. As shown in Fig. 2, in the case body 20, a support part 22 that supports an end part of the assay strip 1 including the absorption pad 6 is formed at a position facing the absorption pad 6. A second accommodating part 32 of the multifunctional member 30 is disposed above the absorption pad 6. The support part 22 also supports the multifunctional member 30 via the absorption pad 6. In addition, in the case body 20, a support part 21 that supports a central part of the assay strip 1 is formed.

### <Assay strip>

The assay strip 1 includes a carrier 2, a liquid feeding pad 4, and an absorption pad 6. Then, the carrier 2 is fixedly supported on a back pressure-sensitive adhesive sheet 7.

### (Carrier)

The carrier 2 is a porous insoluble carrier for developing a sample, and includes an assay region L1, a control region L2, and a color development region L3. In addition, the carrier 2 includes a label holding pad 3. The label holding pad 3 constitutes a spotting region on which the sample is spotted from dropping port 16. The color development region L3 is disposed on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample in the carrier 2.

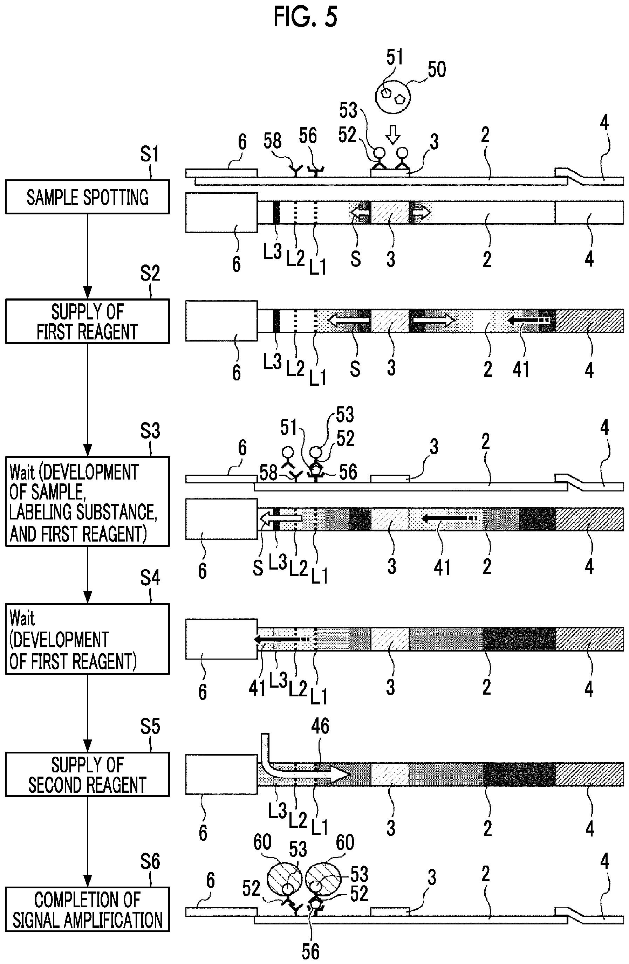

It shows a state in which the assay region L1, the control region L2, and the color development region L3 are expressed as lines, but these are not always expressed. Details will be described later, but before developing the sample 50 (see Fig. 5), the first reagent 41 (see Fig. 4), and the second reagent 46 (see Fig. 4), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by increasing the color optical density in a case where the sample 50 is developed and the developed sample 50 is positive. Since the color development of the assay region L1 is amplified by silver amplification, which will be described later, the assay region L1 develops a black color.

The control region L2 is also expressed as a line by increasing the color optical density in a case where the sample 50 is developed. Accordingly, the control region L2 is visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first reagent 41 is developed. However, the color development region L3 is expressed as an orange line by changing a dark green color to an orange color in a case where the first reagent 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

### (Carrier - label holding pad)

As shown in Fig. 5, a labeling substance 53 is fixed to the label holding pad 3. The labeling substance 53 is modified with the first binding substance 52 that specifically binds to the test substance 51 contained in the sample 50. The label holding pad 3 is fixed on the carrier 2 at a position facing the dropping port 16 (see Fig. 2) of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

### (Carrier - assay region)

As shown in Fig. 5, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bonded to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51.

### (Carrier - control region)

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

### (Carrier - color development region)

The color development region L3 contains a substance whose color development state changes in response to the first reagent 41. The color development region L3 indicates that the first reagent 41 has been developed to that region by reacting with the first reagent 41 to develop a color or change a color. For example, in a case where a mixed aqueous solution of an iron nitrate aqueous solution and citric acid (manufactured by Fujifilm Wako Pure Chemical Corporation, 038-06925) is used as the first reagent 41, an aspect in which the color development region L3 is constituted of a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first reagent 41, and the dark green color is changed to an orange color in a case where the first reagent 41 reaches the color development region L3. The color development region L3 is sometimes referred to as an amplification index region because the timing of supplying the second reagent 46 after the first reagent 41 is developed is indicated by changing the color development state.

### (Binding substance)

The first binding substance 52 that modifies the labeling substance 53 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The second binding substance 56 that is fixed in the assay region L1 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an influenza A virus or a biomarker thereof, anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

### (Liquid feeding pad)

The liquid feeding pad 4 is disposed in contact with one end of the carrier 2 and the first reagent 41 is fed to the carrier 2 from the upstream side of the spotting region (constituted of the label holding pad 3). In the liquid feeding pad 4, in a case where the first pressing operation part 11 is pressed, one end of the liquid feeding pad 4 is immersed in the first reagent holding part 40. The liquid feeding pad 4 is formed of a porous material and absorbs the first reagent 41, and the absorbed first reagent 41 is fed to the carrier 2 by a capillary action.

### (Absorption pad)

The absorption pad 6 is disposed in contact with the other end of the carrier 2 and absorbs the sample 50, the first reagent 41, and the second reagent 46, which are developed on the carrier 2. The absorption pad 6 is also formed of a porous material.

### <Amplifying liquid>

In the present embodiment, the first reagent 41 and the second reagent 46 are amplifying liquids that amplify the color development in the assay region L1 and the control region L2 by reacting with each other. In a case where a metal-based labeling substance such as a gold colloid is used as the labeling substance 53 as in the present example, for example, silver amplification is used as a method of amplifying the labeling signal of the labeling substance 53. The first reagent 41 and the second reagent 46 are, as an example, amplifying liquids used for silver amplification, and the reaction between the first reagent 41 and the second reagent 46 using the labeling substance 53 as a catalyst is an amplification reaction. By the amplification reaction, silver particles having a particle diameter relatively larger than that of the labeling substance 53 are generated.

More specifically, in the present example, the first reagent 41 is a reducing agent that reduces silver ions, and the second reagent 46 is a silver ion. In a case where the first reagent 41, which is a reducing agent, and the second reagent 46, which is a silver ion, are brought into contact with the labeling substance 53, silver particles 60 (see Fig. 5) are generated, and the generated silver particles 60 deposits on the labeling substance 53 using the labeling substance 53 as a nucleus. By depositing the silver particles on the labeling substance 53, silver particles 60 having a particle diameter larger than that of the labeling substance 53 (see Fig. 5) are generated. Accordingly, the labeling signal issued by the labeling substance 53 is amplified, and as a result, the color development of the labeling substance 53 is amplified in the assay region L1 and the control region L2.

### (First Reagent)

As the reducing agent as the first reagent 41, any inorganic or organic material or a mixture thereof can be used as long as the silver ion used as the second reagent 46 can be reduced to silver. Preferred examples of the inorganic reducing agent include a reducing metal salt and a reducing metal complex salt, of which the atomic valence is capable of being changed with a metal ion such as Fe²⁺, V²⁺, or Ti³⁺. In a case where an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of Fe²⁺ is preferably used.

It is also possible to use a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

### (Second reagent)

The solution containing silver ions, which is used as the second reagent 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

### <Immunochromatographic method>

An immunochromatographic method will be described with reference to Fig. 5. Here, a case where the sample 50 includes the test substance 51, that is, on the premise that the sample 50 is positive will be described.

First, the sample 50 is spotted on the label holding pad 3 which is the spotting region (Step S 1). The test substance 51 in the sample 50, which is spotted on the label holding pad 3, specifically binds to the first binding substance 52 that modifies the labeling substance 53 contained in the label holding pad 3. The sample 50 is developed on the downstream side from the label holding pad 3 in the carrier 2 by the capillary action in the carrier 2. Apart of the sample 50 is also developed on the upstream side.

Next, the first reagent 41 is supplied (Step S2). The first reagent 41 is supplied from the liquid feeding pad 4. The first reagent 41 is supplied to the carrier 2 via the liquid feeding pad 4 and is developed on the downstream side.

After that, the process waits until the first reagent 41 is developed on the downstream side (Step S3 and Step S4). "Wait" shown in Fig. 5 means an action of waiting. The first reagent 41 is gradually developed to the downstream side, and the sample 50 to be developed from the label holding pad 3 and the labeling substance 53 modified with the first binding substance 52 are developed to the downstream side to be pushed by the first reagent 41 (Step S3).

The test substance 51 in the sample 50 that has been developed to the downstream side and has reached the assay region L1 is captured by the second binding substance 56 of the assay region L1. That is, the labeling substance 53 is captured in the assay region L1 via the test substance 51 and the first binding substance 52. On the other hand, the labeling substance 53 that is not bound to the test substance 51 passes through the assay region L1 without being captured and is captured by the third binding substance 58 of the control region L2.

In a case where the development of the first reagent 41 proceeds and the first reagent 41 reaches the color development region L3 (Step S4), the color development region L3 reacts with the first reagent 41 to change the color development state. In the present example, the color development region L3 is dark green color before reacting with the first reagent 41, and the dark green color is changed to an orange color by reacting with the first reagent 41.

After the first reagent 41 is sufficiently developed, the second reagent 46 is supplied to the carrier 2 (Step S5). The second reagent 46 is supplied to the carrier 2 from the downstream side of the color development region L3 and is developed on the upstream side. Here, the first reagent 41 is a first amplifying liquid containing a reducing agent that reduces silver ions, and the second reagent 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

The second reagent holding part 45 of the cartridge 100 and a structure around the second reagent holding part 45 will be described with reference to Fig. 6 and below.

### (Second reagent holding part)

As shown in Fig. 6A, the second reagent holding part 45 is an example of a reagent container supplied to the carrier 2. The second reagent holding part 45 of the present example is an enclosing body in which the second reagent 46 supplied to the carrier 2 is enclosed. Fig. 6A shows a state in which the pressing force is not applied to the second reagent holding part 45, and Fig. 6B shows a state in which the pressing force is applied to the second reagent holding part 45. As shown in Fig. 6B, the second reagent holding part 45 has a sealing portion 45A that opens earlier than other portions in a case where the internal pressure is increased due to the application of the pressing force. The second reagent 46 discharged from the second reagent holding part 45 flows out to the supply port 32A by opening the sealing portion 45A.

### (Form and manufacturing method of second reagent holding part)

Fig. 7A to Fig. 7E show an example of a form and a manufacturing method of the second reagent holding part 45. The second reagent holding part 45 of the present example is formed of one sheet member 45S that is rolled into a cylindrical shape. In order to manufacture the second reagent holding part 45, first, the sheet member 45S shown in Fig. 7A is prepared and rolled into a cylindrical shape as shown in Fig. 7B.

As the sheet member 45S, a film formed of any material chemically inactive to the second reagent 46 can be used. Specifically, a film formed of a general-purpose resin such as polypropylene, polyethylene, polyethylene terephthalate, or an Acrylonitrile-Butadiene-Styrene (ABS) resin can be used. In addition, it is more preferable to use a composite film in which an aluminum layer for preventing permeation of moisture or gas is added to the resin layer formed of these resins.

Next, the end portions 45C1 and 45C2 of the rolled sheet member 45S along the cylinder axis direction are welded to each other as shown in Fig. 7C to form the sealing portion 45C.

Next, as shown in Fig. 7D, one end portion in the cylinder axis direction is welded along a direction intersecting the cylinder axis direction to form the sealing portion 45A. Then, the second reagent 46 is filled inside sheet member 45S from the end portion on the opposite side of the sealing portion 45A.

Next, as shown in Fig. 7E, the sealing portion 45B is formed by welding the end portion on the opposite side of the sealing portion 45A. Accordingly, the second reagent holding part 45 is formed as an enclosing body in which the second reagent 46 is enclosed therein.

The second reagent holding part 45 of the present example is manufactured by processing one sheet member 45S into a cylindrical shape in this manner. Such a form of the second reagent holding part 45 is generally called a pillow packaging body or the like. Therefore, the second reagent holding part 45 may be manufactured using a pillow packaging machine or the like.

In this way, the second reagent holding part 45 is formed from one sheet member 45S, and thus the number of locations of the sealing portion can be reduced as compared with a case where the second reagent holding part 45 is formed from a combination of two or more sheets. Accordingly, the number of steps involved in processing can be reduced.

In the second reagent holding part 45, sealing portions 45A and 45B are formed at both end portions in the longitudinal direction. Among these, the sealing portion 45A is a sealing portion disposed at the supply port 32A in a state where the second reagent holding part 45 is disposed at the second accommodating part 32. In addition, the sealing portion 45B is a portion disposed on the side opposite to the supply port 32A in a state where the second reagent holding part 45 is disposed in the second accommodating part 32.

Here, the "sealing portion disposed at the supply port" in the present disclosure refers to the sealing portion 45A disposed at a position closer to the supply port 32A than other portions in the second reagent holding part 45. Another example of the "sealing portion disposed at the supply port" will be described later.

The sealing portions 45A and 45B are sealing portions that seal the second reagent holding part 45 to prevent the leakage of the second reagent 46 from the second reagent holding part 45 in a state where the pressing force does not apply to the second reagent holding part 45, in other words, in a state where an external force other than the atmospheric pressure does not apply to the second reagent holding part 45. In addition, in the present example, the sealing portion 45A is a sealing portion that opens earlier than the other portions in a case where the internal pressure of the second reagent holding part 45 is increased due to the application of the pressing force to the second reagent holding part 45.

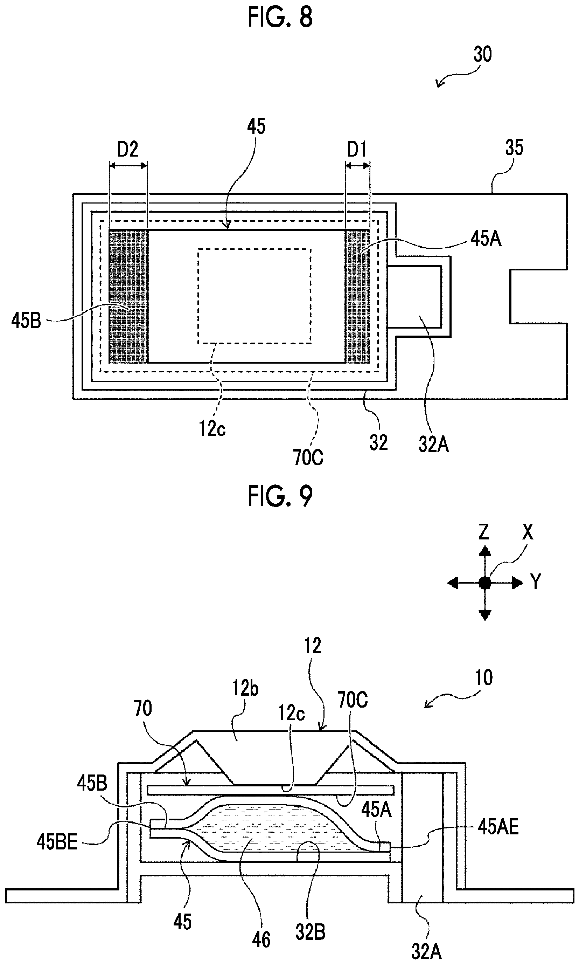

The sealing portions 45A and 45B are formed by heat-welding the sheet member 45S. That is, the sealing portions 45A and 45B are heat-welded portions. As shown in Fig. 8, the welding width D1 of the sealing portion 45A is smaller than the welding width D2 of the sealing portion 45B. Therefore, the welding area of the sealing portion 45A is smaller than the welding area of the sealing portion 45B. On the other hand, the temperature, time, and pressure for welding the sealing portions 45A and 45B are the same. Accordingly, the sealing portion 45A is a weak sealing portion having a weak sealing force as compared with the sealing portion 45B. Therefore, in the present example, the sealing portion 45A opens earlier than the other portions in a case where the internal pressure of the second reagent holding part 45 is increased.

As shown in Fig. 6, the second reagent holding part 45 is accommodated inside the second accommodating part 32, and a second displacement part 70 is disposed between the second reagent holding part 45 and the first displacement part 12b. The second reagent holding part 45 is accommodated in a state of abutting on the second displacement part 70. In addition, as shown in Fig. 8 in which the second accommodating part 32 is viewed in a plan view from the pressing direction (Z direction) in which the second reagent holding part 45 is pressed, the second reagent holding part 45 is disposed along a longitudinal direction as a Y direction (longitudinal direction of the assay strip 1, see Fig. 2 and the like) in a state of being disposed inside the second accommodating part 32.

In the second accommodating part 32, the supply port 32A is provided in the upstream side close to the assay region L1. Accordingly, for example, as compared with a case where the supply port 32Ais provided on the downstream side of the second accommodating part 32, the second reagent 46 discharged from the supply port 32A is rapidly supplied to the assay region L1 of the carrier 2.

In addition, the supply port 32A is formed to be narrower in width than the second accommodating part 32 and is provided in the central part of the second accommodating part 32. The "width" is a width along the lateral direction (X direction) of the case body 20, and the "central part" is a center part of the case body 20 in the lateral direction (X direction). This central part is a location located above the carrier 2. Accordingly, the second reagent 46 discharged from the supply port 32A is rapidly fed to the carrier 2.

As shown in Fig. 6, the bottom surface 32B of the second accommodating part 32 abuts on the second reagent holding part 45, and the second reagent holding part 45 abuts on the second displacement part 70. The second displacement part 70 is an intermediate member disposed between the first displacement part 12b integrally formed with the second pressing operation part 12 and the second reagent holding part 45. In the pressing direction (Z direction) in which the second reagent holding part 45 is pressed, the thickness H2 of the second displacement part 70 is formed to be smaller than the thickness H1 of the first displacement part 12b.

In addition, the second displacement part 70 includes a second pressing surface 70C. The second pressing surface 70C is a pressing surface that crushes the second reagent holding part 45 in a case where the second displacement part 70 is pressed by the first pressing surface 12c of the first displacement part 12b. In addition, in the second displacement part 70, the second pressing surface 70C that faces and abuts on the second reagent holding part 45 is a smooth flat surface having little unevenness. The second pressing surface 70C also functions as a pressing part that applies a pressing force to the second reagent holding part 45 in a case where the second pressing operation part 12 is operated. The bottom surface 32B of the second accommodating part 32 also abuts on the second reagent holding part 45 on the side opposite to the second pressing surface 70C. The bottom surface 32B is also a flat surface similarly to the second pressing surface 70C.

In addition, in Fig. 8, the outlines of the first pressing surface 12c and the second pressing surface 70C are shown by broken lines. In the present example, the projected area of the second pressing surface 70C viewed from the pressing direction in which the second reagent holding part 45 is pressed is larger than the first pressing surface 12c. In addition, the projected area of the second pressing surface 70C viewed from the pressing direction in which the second reagent holding part 45 is pressed is larger than the projected area of the second reagent holding part 45.

### <Action and effect>

### (Effects of second reagent holding part and second accommodating part)

As shown in Fig. 6A, the cartridge 100 of the present disclosure includes a second reagent holding part 45 as an enclosing body in which the second reagent 46 is enclosed. The second reagent holding part 45 has a sealing portion 45A that opens earlier than the other portions in a case where the internal pressure is increased due to the application of the pressing force.

According to this configuration, as shown in Fig. 6B, the second reagent 46 is discharged to the outside of the second reagent holding part 45 from the sealing portion 45A that opens earlier, and thus it is easy to regulate the discharge direction of the second reagent 46 as compared with the configuration in which the second reagent holding part 45 is broken by the protrusions as in the prior art. In a case where the discharge direction of the second reagent 46 can be regulated, the generation of a wasted second reagent 46 that is not supplied to the carrier 2 (see Fig. 2, Fig. 3A, and Fig. 3B) can be suppressed.

In addition, in the cartridge 100 of the present disclosure, the second pressing surface 70C of the second displacement part 70 is a smooth flat surface having little unevenness and is a pressing part that applies the pressing force to the second reagent holding part 45 in a case where the second pressing operation part 12 is operated.

Since the pressing force is applied to the second reagent holding part 45 by such a flat surface, the pressing force is less likely to be locally concentrated as compared with the configuration in which the second reagent holding part 45 is broken by the protrusions as in the prior art, and the breakage of portions other than the sealing portion 45A can be suppressed.

In addition, in the cartridge 100 of the present disclosure, the second accommodating part 32 for accommodating the second reagent holding part 45 that is an enclosing body is provided in the case 9 formed of the cover member 10 and the case body 20. In addition, in the second accommodating part 32, the supply port 32A for supplying the second reagent 46 discharged from the second reagent holding part 45 to the carrier 2 is formed. Furthermore, in the second reagent holding part 45, the sealing portion 45A is disposed at the supply port 32A.

Accordingly, the second reagent 46 discharged from the sealing portion 45A easily flows into the supply port 32A in which the sealing portion 45A is disposed. Therefore, wasted reagents flowing to other than the supply port 32A are reduced.

On the other hand, for example, in a case where the sealing portion 45A is disposed at a position distant from the supply port 32A, the second reagent 46 discharged from the sealing portion 45A flows toward the supply port 32A in the second accommodating part 32. In this case, liquid residue may be generated in a portion between the sealing portion 45A and the supply port 32A. In addition, in order to suppress the liquid residue, it may be necessary to provide a gradient for flowing the second reagent 46 to the supply port 32A on the bottom surface 32B of the second accommodating part 32, and the configuration of the second accommodating part 32 is complicated.

In addition, in the cartridge 100 of the present disclosure, the second reagent holding part 45 is provided with a plurality of sealing portions, that is, the sealing portions 45A and 45B. The sealing portion 45A, which is one of the plurality of sealing portions 45A and 45B, is a weak sealing portion having a weaker sealing force than the other sealing portion 45B. Accordingly, the second reagent 46 is discharged from the sealing portion 45A, which is a weak sealing portion, and thus easily flows into the supply port 32A. Therefore, the wasted second reagent 46 flowing to other than the supply port 32Ais reduced.

In the present embodiment, between the sealing portions 45A and 45B, the sealing portion 45A disposed at the supply port 32A is a weak sealing portion, but the embodiments of the present disclosure are not limited to this. For example, the welding areas of the sealing portions 45A and 45B may be equal each other to equalize the sealing forces of the two sealing portions 45A and 45B.

In this case, the sealing portions 45A and 45B are opened at the same time, or one of the sealing portions 45A and 45B is opened first, but in a case where at least any one of the sealing portions 45A and 45B opens earlier than the other portions of the second reagent holding part 45, it is easy to regulate the direction in which the second reagent 46 is discharged. In addition, for example, the second reagent holding part 45 is disposed in a posture where the cylinder axis direction connecting the sealing portions 45A and 45B intersects the longitudinal direction of the carrier 2, and the distance between each of the sealing portions 45A and 45B and the supply port 32A may be the same. That is, it is an aspect in which the second reagent holding part 45 is disposed in a posture of being lateral orientation with respect to the longitudinal direction of the carrier 2. Thus, since the two sealing portions 45A and 45B are disposed on both sides of the carrier 2 in the longitudinal direction, the distances between the sealing portions and the supply port 32A located immediately above the carrier 2 are equal. In this case, even in a case where both the sealing portions 45A and 45B are opened, the second reagent 46 discharged from both can be flowed to the supply port 32A.

In addition, in the cartridge 100 of the present disclosure, the sealing portions 45A and 45B are welded. Accordingly, for example, as compared with a case of sealing with a tape, it is easy to manufacture and there is less risk of liquid leakage. In addition, these sealing portions 45A and 45B are heat-welded. Heat-welding is more general as a sealing method of an enclosing body such as the second reagent holding part 45, and thus is easy to prepare. In addition, since it is not necessary to use an adhesive or the like other than the material forming the second reagent holding part 45 for sealing, it is possible to reduce an adverse effect due to a chemical reaction of such an adhesive with the second reagent 46.

In addition, in the cartridge 100 of the present disclosure, the sealing portion 45A is a weak sealing portion because the welding area is smaller than that of the sealing portion 45B. The method of adjusting the sealing force with the welding area is simple because it is not necessary to adjust the welding temperature, the welding pressure, and the welding time between the plurality of sealing portions. Therefore, the sealing force can be easily adjusted as compared with the case where the welding temperature, the welding pressure, the welding time, and the like are adjusted.

In addition, in the cartridge 100 of the present disclosure, as a method of forming the weak sealing portion, a method other than the method of adjusting the welding area can also be adopted. For example, the weak sealing portion may be formed by controlling the thermal energy applied to the second reagent holding part 45. As a method of controlling the thermal energy, there is a method of adjusting a welding temperature, a welding pressure, a welding time, or the like.

For example, in a case where the welding temperature is set to be lower than the welding temperature of the other sealing portions, the amount of melting of the sheet member 45S, which is a material for forming the second reagent holding part 45, is reduced, and thus a weak sealing portion can be formed. In addition, in a case where the welding pressure is set to be lower than the welding pressure of the other sealing portions, the degree of adhesion between the melting parts is lowered, and thus a weak sealing portion can be formed. Furthermore, even in a case where the welding time is shortened, the amount of melting of the material forming the second reagent holding part 45 is reduced, and thus the weak sealing portion can be formed.

In a case where the weak sealing portion is formed by adjusting the welding temperature, the welding pressure, the welding time, or the like instead of the welding area, a plurality of welding areas can be made equal. That is, it is not necessary to make the area of the sealing portion other than the weak sealing portion larger than the area of the weak sealing portion. Accordingly, the second reagent holding part 45 and the second accommodating part 32 can be miniaturized.

In this way, in a case where the method of sealing the sealing portions 45A and 45B by heat-welding is adopted, the number of adjustment parameters such as the welding temperature, the welding pressure (pushing pressure of the heating element), the welding time, and the welding area is relatively large, and thus it is easy to adjust the sealing force.

In the present embodiment, heat-welding is used as the welding method of the sealing portions 45A and 45B, but the embodiments of the present disclosure are not limited to this. For example, as a welding method of the sealing portions 45A and 45B, high-frequency welding, ultrasonic welding, or the like may be adopted. In addition, welding can also be performed using a solvent chemically inactive to the second reagent 46.

In the present embodiment, as shown in Fig. 6A, the positions of the sealing portions 45A and 45B are not fixed in the second accommodating part 32, but the embodiments of the present disclosure are not limited to this. For example, as shown in Fig. 9, the position of the sealing portion 45A disposed at the supply port 32A may be fixed. As a direction for fixing the position of the sealing portion 45A, for example, there is a method of fixing the sealing portion 45A to the bottom surface 32B of the second accommodating part 32 with a double-sided tape or the like.

In this way, in a case where the position of the sealing portion 45A is fixed, it is possible to prevent misregistration of the sealing portion 45A due to the floating of the sealing portion 45A or the like in a case where the second reagent holding part 45 is pressed, and to suppress the sealing portion 45A from being separated from the supply port 32A.

In the example shown in Fig. 9, only the sealing portion 45A is fixed to the bottom surface 32B of the second accommodating part 32, but not only the sealing portion 45A but also the sealing portion 45B may be fixed to the bottom surface 32B, and a portion other than the sealing portions 45A and 45B may be fixed to the bottom surface 32B.

In addition, in the second reagent holding part 45 shown in Fig. 6A and Fig. 9, the sealing portion 45A is disposed at a position close to the supply port 32A, but the embodiments of the present disclosure are not limited to this. For example, the sealing portion 45A may be connected to the supply port 32A via the "connecting portion".

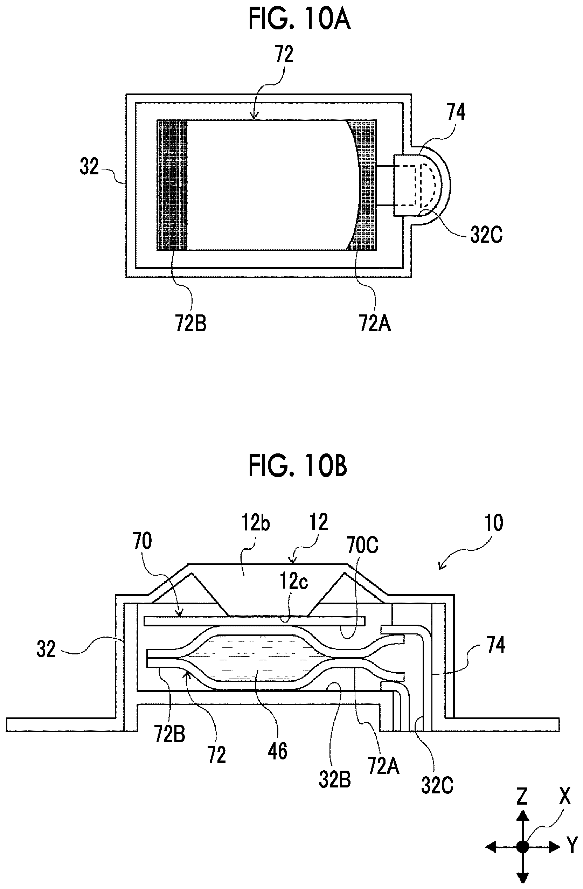

As an example of the second reagent holding part provided with the connecting portion, Fig. 10A shows the second reagent holding part 72. Fig. 10A is a plan view of the second reagent holding part 72 viewed from the Z direction, which is the pressing direction, and Fig. 10B is a cross-sectional view viewed from the X direction. The second reagent holding part 72 shown in Fig. 10A also includes two sealing portions 72A and 72B, and the sealing portion 72A is a weak sealing portion having a weaker sealing force than the sealing portion 72B. The tubular body 74, which is a member separate from the second reagent holding part 72, is connected to the sealing portion 72A as a connecting portion.

In this way, the sealing portion 72A is disposed at the supply port 32C via the tubular body 74 as the connecting portion in the second accommodating part 32, and thus the second reagent 46 discharged from the sealing portion 72A is easy to flow into the supply port 32C. Accordingly, the wasted second reagent 46 flowing to other than the supply port 32C is reduced. In addition, the sealing portion 72A is connected to the supply port 32C via the tubular body 74, and thus the degree of freedom in the arrangement of the sealing portion 72A is increased.

In addition, by using a tubular body different from the second reagent holding part 72 as the connecting portion, the shape and material of the connecting portion can be freely selected. Accordingly, an appropriate connecting portion can be provided in consideration of the shape of the second accommodating part 32, the shape of the supply port 32C, and the shape, the material, and the like of the second reagent holding part 72.

For example, the tubular body 74 is formed of a material that is harder than the material of the sheet member 45S that forms the second reagent holding part 72, and is inserted into the supply port 32A. Accordingly, the flow of the second reagent 46 is stabilized. Then, the tubular body 74 and the supply port 32C are formed such that the outer peripheral surface of the tubular body 74 and the inner peripheral surface of the supply port 32C are in contact with each other. Accordingly, in a state where the tubular body 74 is inserted into the supply port 32C, misregistration is less likely to occur. As the tubular body 74, similarly to the sheet member 45S, any material that is chemically inactive to the second reagent 46 can be used. Specifically, a film formed of a general-purpose resin such as polypropylene, polyethylene, polyethylene terephthalate, or an Acrylonitrile-Butadiene-Styrene (ABS) resin can be used. In addition, it is more preferable to use a composite film in which an aluminum layer for preventing permeation of moisture or gas is added to the resin layer formed of these resins.

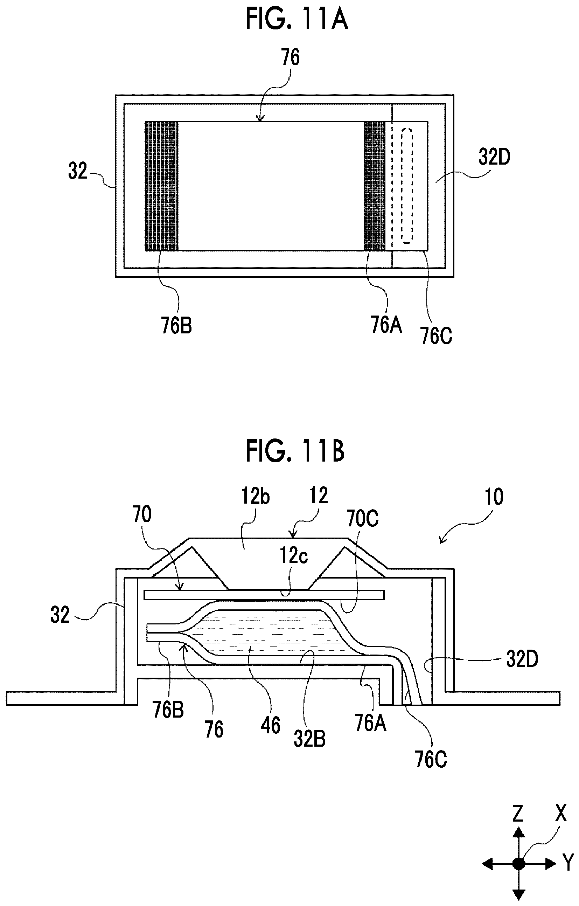

In addition, as another example of the second reagent holding part provided with the connecting portion, the second reagent holding part 76 is shown in Fig. 11A and Fig. 11B. Fig. 11A is a plan view of the second reagent holding part 76 viewed from the Z direction, and Fig. 11B is a cross-sectional view viewed from the X direction. The second reagent holding part 76 also includes two sealing portions 76A and 76B, and the sealing portion 76A is a weak sealing portion having a weaker sealing force than the sealing portion 76B. A connecting portion 76C is connected to the sealing portion 76A. The connecting portion 76C is a part of the second reagent holding part 76, has the same width as the second reagent holding part 76, and is a portion extended in the longitudinal direction of the second reagent holding part 76.

In a state in which the second reagent holding part 76 is accommodated in the second accommodating part 32, the connecting portion 76C is extended from the sealing portion 76B toward the supply port 32D and is further inserted into the supply port 32D. The supply port 32D has a width equal to that of the second accommodating part 32 in the lateral direction (X direction) of the case body 20 such that the connecting portion 76C is inserted, and has a wide opening portion as compared with that of the supply port 32A shown in Fig. 9.

By using a part of the second reagent holding part as the connecting portion as in the connecting portion 76C, the number of parts is small and the cost is low for preparing the second reagent holding part, as compared with the case where the connecting portion is a separate member from the second reagent holding part. In addition, the connecting portion 76C is formed to have the same width as the second reagent holding part 76 and is further inserted into the wide supply port 32D, and thus the second reagent 46 is easily discharged from the supply port 32D.

Not only the connecting portion 76C but also the sealing portion 76A may be inserted into the supply port 32D. In this way, the "sealing portion disposed at the supply port" in the present disclosure includes the sealing portion 76A or the like inserted into the supply port 32D.

In addition, in the present example, the second reagent holding part 45 in which the connecting portion is "not formed" as shown in Fig. 6A, may be disposed in the second accommodating part 32 in which the supply port 32D is formed to be wide. Also in this case, the supply port 32D is formed to be wide, and thus an effect that the second reagent 46 is easily discharged from the supply port 32D can be obtained. In this time, the sealing portion 45A of the second reagent holding part 45 may be fixed to the bottom surface 32B or may be disposed to face the supply port 32D. In this way, the "sealing portion disposed at the supply port" in the present disclosure includes the sealing portion 45A in a state of facing the supply port 32D.

### (Effects of first displacement part and second displacement part)

As shown in Fig. 6B, in the cartridge 100 of the present disclosure, the second reagent holding part 45 is crushed in a case where a pressing force is applied, to discharge the second reagent 46 to the outside. In addition, the cartridge 100 includes a first displacement part 12b and a second displacement part 70 that can be displaced in the case 9.

The first displacement part 12b has the first pressing surface 12c for pushing the second displacement part 70 to the second reagent holding part 45 by pressing the second displacement part 70 in a case where the second pressing operation part 12 which is a pressing operation part is operated.

Then, the second displacement part 70 is disposed between the first displacement part 12b and the second reagent holding part 45, and has a second pressing surface 70C that crushes the second reagent holding part 45 in a case where the second displacement part 70 is pressed from the first pressing surface 12c. As shown in Fig. 8, the projected area of this second pressing surface 70C viewed from the pressing direction (Z direction) in which the second reagent holding part 45 is pressed is larger than the first pressing surface 12c.

Here, since the size of the first displacement part 12b, which is closer to the second pressing operation part 12 (integrated with the second pressing operation part 12 in the present embodiment), may be relatively small, it is easy to secure the movable range of the second pressing operation part 12. On the other hand, the area of the second pressing surface 70C of the second displacement part 70, which is closer to the second reagent holding part 45, can be relatively large.

That is, since the size of the first displacement part 12b may be relatively small, it is easy to secure the movable range of the second pressing operation part 12. In addition, since the second pressing surface 70C of the second displacement part 70 can be relatively wide, a relatively wide range of the second reagent holding part 45 can be crushed. Therefore, it is possible to suppress the liquid residue in which the second reagent 46 remains inside the second reagent holding part 45.

In addition, in the cartridge 100 of the present disclosure, in a case where in the second reagent holding part 45, a sealing portion 45A side is defined as a first end portion 45AE, an end portion on a side opposite to the sealing portion 45A is defined as a second end portion 45BE, and a direction in which the first end portion 45AE and the second end portion 45BE are connected is defined as a reference direction (a direction along the Y direction), in this reference direction, the width of the second pressing surface 70C is larger than the width of the first pressing surface 12c of the first displacement part 12b.

In order to squeeze out the second reagent 46 from the second end portion 45BE side toward the first end portion 45AE side where the sealing portion 45A is located, the second reagent holding part 45 is preferably pressed over a wide range in the reference direction. In a case where the width of the second pressing surface 70C is larger than the width of the first pressing surface 12c in the reference direction, the liquid residue of the second reagent 46 is further suppressed. In addition, since the increase in size of the first pressing surface 12c is suppressed, it is easy to secure a movable range of the second pressing operation part 12.

In the above-described reference direction, the width of the second pressing surface 70C may be narrower than the width of the first pressing surface 12c of the first displacement part 12b. Also in this case, for example, by setting the width of the second pressing surface 70C to be larger than the width of the first pressing surface 12c of the first displacement part 12b in the direction (X direction) intersecting the reference direction, the projected area of the second pressing surface 70C of the second displacement part 70 just needs to be larger than the projected area of the first pressing surface 12c of the first displacement part 12b. In a case where the projected area of the second pressing surface 70C of the second displacement part 70 is formed to be larger than the projected area of the first pressing surface 12c of the first displacement part 12b, it is possible to obtain an effect of suppressing liquid residue in which the second reagent 46 remains inside the reagent holding part 45 as compared with a case where the projected area is narrow.

In addition, in the cartridge 100 of the present disclosure, as shown in Fig. 8, the projected area of the second pressing surface 70C is larger than the projected area of the second reagent holding part 45. Therefore, the second displacement part 70 can press the entire area of the second reagent holding part 45. Accordingly, the liquid residue of the second reagent 46 inside the second reagent holding part 45 can be further suppressed.

In addition, in the cartridge 100 of the present disclosure, as shown in Fig. 6A, Fig. 6B, Fig. 10A, Fig. 10B, Fig. 11A, and Fig. 11B, the thickness H2 of the second displacement part 70 is narrower than the thickness H1 of the first displacement part 12b in the pressing direction in which the second reagent holding part 45 is pressed. By setting the thickness of the second displacement part 70 to be narrower in this manner, it is easy to secure a movable range of the second pressing operation part 12.

In addition, in the cartridge 100 of the present disclosure, as shown in Fig. 6A, Fig. 6B, Fig. 10A, Fig. 10B, Fig. 11A, and Fig. 11B, the second pressing operation part 12 and the first displacement part 12b are integrally formed. Accordingly, the number of parts of the cartridge 100 can be reduced as compared with a configuration in which the second pressing operation part 12 and the first displacement part 12b are constituted of separate members.

In the cartridge 100 of the present disclosure, as shown in Fig. 6A, Fig. 6B, Fig. 10A, Fig. 10B, Fig. 11A, and Fig. 11B, in a case where the second displacement part 70 is pressed by the first displacement part 12b, the second pressing surface 70C evenly presses the second reagent holding part 45, that is, presses the second reagent holding part 45 such that the amount of sinking between the first end portion 45AE side and the second end portion 45BE side is the same, but the embodiments of the present disclosure are not limited to this.

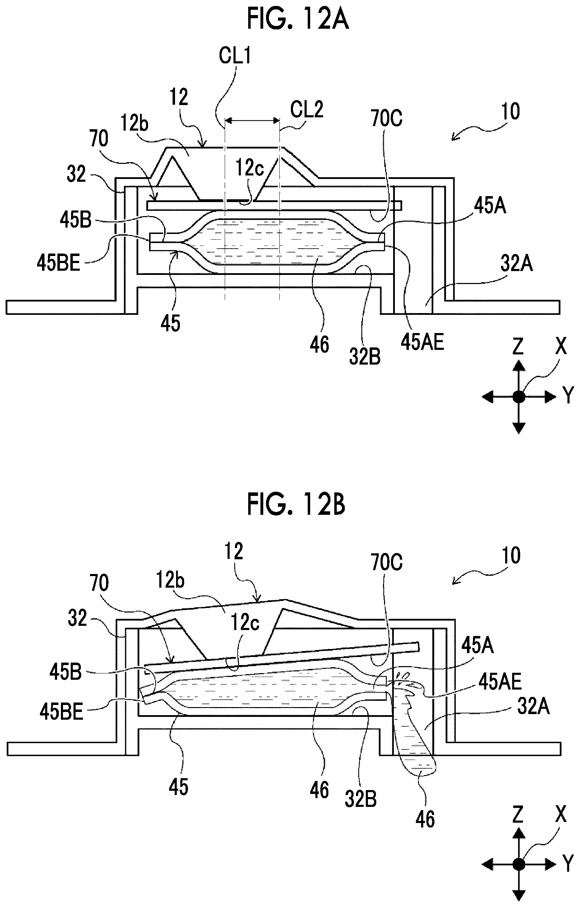

For example, in the second displacement part of the present disclosure, the second end portion 45BE side may be crushed earlier than the first end portion 45AE side of the second reagent holding part 45. An example of such an embodiment, for example, is an aspect shown in Fig. 12A and Fig. 12B.

In this aspect, as shown in Fig. 12A, in the reference direction described above, the center of the first displacement part 12b indicated by the axis CL1 is disposed closer to the second end portion 45BE than the center of the second displacement part 70 indicated by the axis CL2.

Accordingly, as shown in Fig. 12B, the second pressing surface 70C crushes the second end portion 45BE side of the second reagent holding part 45 earlier than the first end portion 45AE side. Accordingly, the second reagent 46 on the second end portion 45BE side of the second reagent holding part 45 can be easily squeezed out to the sealing portion 45A side. Therefore, the liquid residue of the second reagent 46 is suppressed.

In this way, in a simple configuration for adjusting the relative positional relationship between the first displacement part 12b and the second displacement part 70, the second end portion 45BE side can be crushed earlier, and the liquid residue of the second reagent 46 is suppressed.

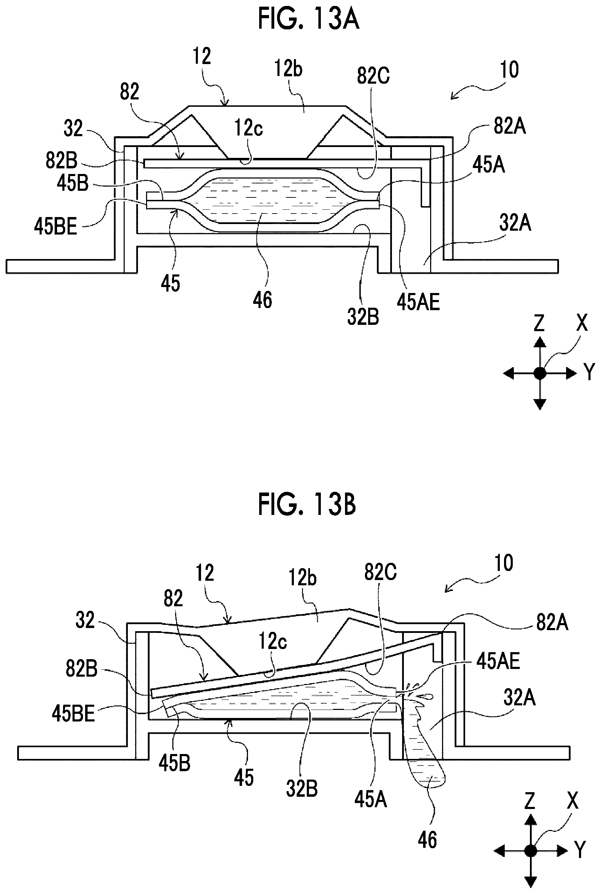

In addition, another example of the embodiment in which the second displacement part crushes the second end portion 45BE side of the second reagent holding part 45 earlier than the first end portion 45AE side is, for example, the aspect shown in Fig. 13A and Fig. 13B.

As shown in Fig. 13A, in the second displacement part 82 used in this embodiment, the end portion 82A on the first end portion 45AE side is defined as a fixed end, and the end portion 82B on the second end portion 45BE side is defined as a free end. Therefore, as shown in Fig. 13B, in a case where the second displacement part 82 is pressed by the first displacement part 12b, the second end portion 45BE side earlier than the first end portion 45AE side of the second displacement part 82 comes into contact with the second reagent holding part 45. Then, the second end portion 45BE side is crushed earlier than the first end portion 45AE side.

The "fixed end" is, for example, an end portion fixed to the inner peripheral surface of the second accommodating part 32 by using an adhesive or the like, and is an end portion that does not release from the second accommodating part 32 due to the pressing force applied from the first displacement part 12b. In addition, the "free end" is an end portion that is not fixed anywhere and is a portion that is easily moved by a pressing force applied from the first displacement part 12b.

In this way, since the end portion 82A of the second displacement part 82 on the first end portion 45AE side is a fixed end and the end portion 82B on the second end portion 45BE side is a free end, the second displacement part 82 can move to be rotated with the end portion 82A on the first end portion 45AE side as a fulcrum.

Then, in a case where the second displacement part 82 is pressed by the first displacement part 12b, the second end portion 45BE side earlier than the fixed first end portion 45AE side of the second displacement part 82 comes into contact with the second reagent holding part 45. Accordingly, the second pressing surface 82C can crush the second end portion 45BE side of the second reagent holding part 45 earlier, and thus the liquid residue of the second reagent 46 is suppressed.

The second displacement part 82 may be formed of a material that is not deformed or is less likely to be deformed other than rotation of the fixed end in a case of being pressed by the first displacement part 12b, but as shown in Fig. 13B, is preferably formed of a deformable material. Accordingly, an area for crushing the second reagent holding part 45 is larger as compared with a case where the second displacement part 82 is not deformed. Therefore, the liquid residue of the second reagent 46 is suppressed.

On the other hand, in a case where the second displacement part 82 is not deformed, it is difficult to transmit the pressing force to the second reagent holding part 45 after the free end on the second end portion 45BE side has moved to the movable limit. As an aspect of the deformation of the second displacement part 82, elastic deformation is preferable. In a case where the second displacement part 82 is plastically deformed, the second displacement part 82 may be cracked. In this case, the broken debris of the second displacement part 82 may inhibit the flow of the second reagent 46. Therefore, the second displacement part 82 is preferably elastically deformed.

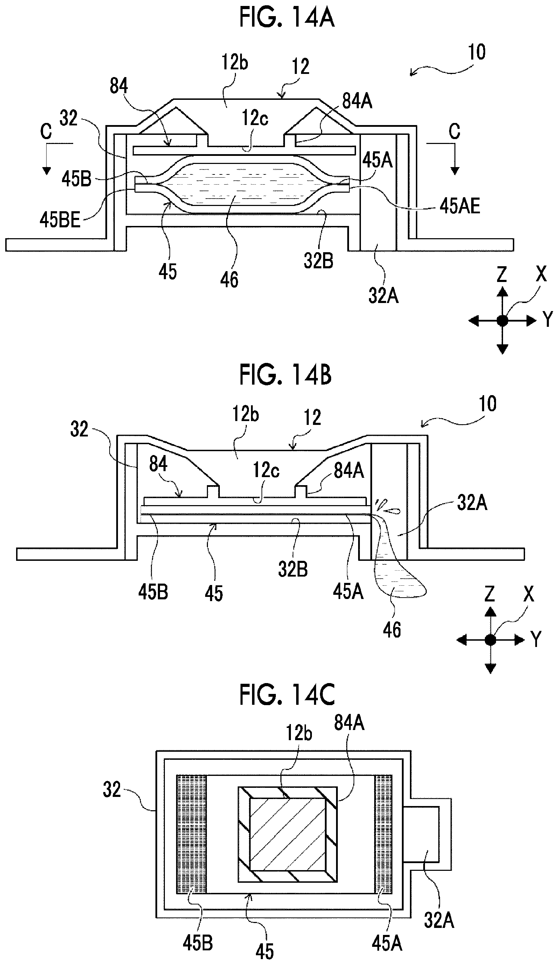

In addition, in the cartridge 100 of the present disclosure, instead of the second displacement part 70, a regulating part 84Athat regulates the position of the first displacement part 12b may be provided similarly the second displacement part 84 shown in Fig. 14A, Fig. 14B, and Fig. 14C. As shown in Fig. 14A and Fig. 14B, the regulating part 84A is a protruding portion that protrudes from the plate-shaped second displacement part 84 toward the first displacement part 12b.

As shown in Fig. 14C, for example, this regulating part 84A is disposed to surround the first displacement part 12b and is fixed to the first displacement part 12b.

In this way, since the first displacement part 12b is positioned with respect to the second displacement part 84, the position where the first displacement part 12b presses the second displacement part 84 is stable. Accordingly, for example, even in a case where a pressing method such as a pressing position and a pressing force with respect to the second pressing operation part 12 is different for each person, the position and the posture in which the second displacement part 84 presses the second reagent holding part 45 as compared with a case where the regulating part 84A is not provided. As a result, even in a case where the pressing method is different, the effect of suppressing the liquid residue can be stably exhibited.

In each of the above-described embodiments, the first displacement part 12b has a trapezoidal shape in a side view as shown in Fig. 6A, and while maintaining this shape, the second displacement part 70 is pressed as shown in Fig. 6B, but the embodiments of the present disclosure are not limited to this.

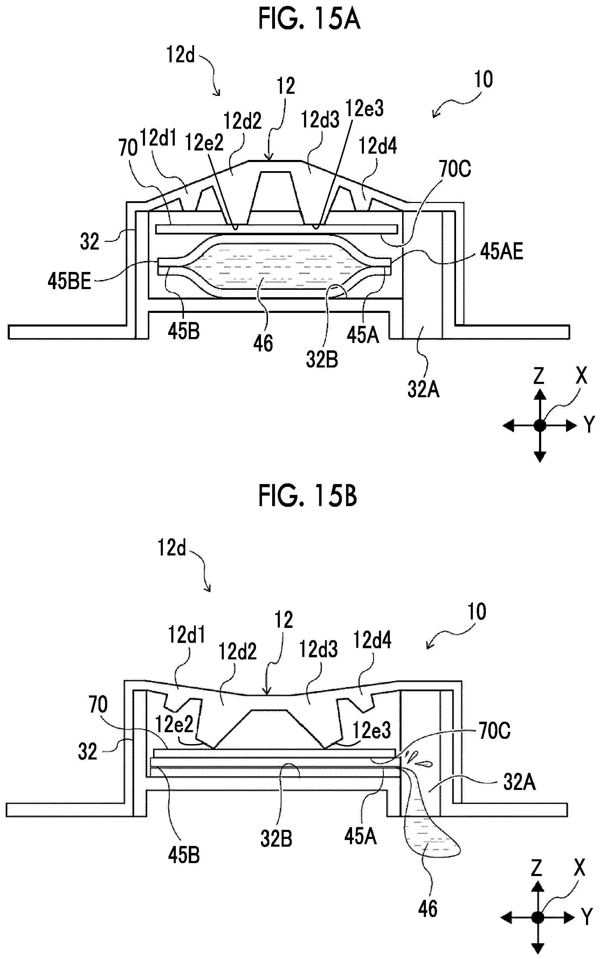

For example, the first displacement part may have a plurality of protrusions 12d1, 12d2, 12d3, and 12d4 as in the first displacement part 12d shown in Fig. 15A and Fig. 15B. As shown in Fig. 15A, a gap is formed between the protrusions. As shown in Fig. 15B, the first displacement part 12d thus formed is deformed such that a gap between the protrusions is opened in a case where the second displacement part 70 is pressed.

In the first displacement part 12d that is deformed in this manner, the first pressing surface is, for example, the pressing surfaces 12e2 and 12e3 that are in contact with the second displacement part 70 in the initial state before the deformation. In this case, the projected area of the first pressing surface is the total area of the pressing surfaces 12e2 and 12e3.

In each of the above-described embodiments, a second displacement part (for example, a second displacement part 70) is provided between the first displacement part (for example, the first displacement part 12b) and the second reagent holding part (for example, the second reagent holding part 45). In a case where the second reagent holding part 45 is used as the enclosing body, the second displacement part may be omitted. In a case where the second displacement part is omitted, but the sealing portion 45A that opens earlier than the other portions in a case where the internal pressure is increased due to the application of the pressing force is formed in the second reagent holding part 45, an effect of suppressing the generation of a wasted reagent that is not supplied to the carrier 2 can be obtained. In addition, in a case where the second displacement part is not provided in this way, the pressing part that applies the pressing force by being pressed against the second reagent holding part 45 as the enclosing body in a case where the second pressing operation part 12 is operated, is the first displacement part 12b of the second pressing operation part 12. It is preferably that the first displacement part 12b includes a first pressing surface 12c as an abutting surface that abuts on the second reagent holding part 45 as an enclosing body, and the first pressing surface 12c is a flat surface.

As described above, in the cartridge 100, the user can visually confirm the color development state of the assay region L1 through the observation window 18. Further, in the cartridge 100, it is also possible to confirm the color development state of the assay region L1 by using an immunochromatographic assay apparatus (hereinafter, simply referred to as an assay apparatus) (not shown). The assay apparatus detects a color development state of the assay region L1 of the loaded cartridge 100, determines whether the sample is positive or negative based on the detected color development state, and presents the determination result. Some such assay apparatus include an internal mechanism that presses a pressing operation part such as a second pressing operation part 12 in a state in which the cartridge 100 is loaded. The internal mechanism is constituted of an actuator such as a solenoid having a movable portion and a motor. In this way, the pressing operation part may be operated by an actuator in addition to or instead of a person. The technique of the present disclosure is effective even in a case where the pressing operation part is operated by the actuator.

The present disclosure is not limited to the above embodiments, and can be implemented with appropriate modifications, such as omitting a configuration or replacing a configuration with a different configuration within the scope that does not deviate from the gist of the present disclosure.

The disclosure of Japanese Patent Application No. 2021-054326 filed on March 26, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

## Claims

1. An assay cartridge used for immunochromatographic assay, comprising:
a carrier to which a sample and a reagent are supplied;
an enclosing body in which the reagent to be supplied to the carrier is enclosed, and which has a sealing portion that opens earlier than other portions in a case where an internal pressure is increased due to an application of a pressing force; and
a case accommodating the carrier and the enclosing body and having a pressing operation part for applying the pressing force to the enclosing body.

2. The assay cartridge according to claim 1, further comprising:
a pressing part that applies the pressing force by being pressed against the enclosing body in a case where the pressing operation part is operated, and in which an abutting surface abutting on the enclosing body is a flat surface.

3. The assay cartridge according to claim 1 or 2,
wherein in the case, an accommodating part that accommodates the enclosing body and in which a supply port for supplying the reagent discharged from the enclosing body to the carrier is formed, is provided, and
in the accommodating part, the sealing portion is disposed at the supply port.

4. The assay cartridge according to claim 3,
wherein a position of the sealing portion disposed at the supply port is fixed.

5. The assay cartridge according to claim 1 or 2,
wherein in the case, an accommodating part that accommodates the enclosing body and in which a supply port for supplying the reagent discharged from the enclosing body to the carrier is formed, is provided, and
in the accommodating part, the sealing portion is connected to the supply port through a connecting portion.

6. The assay cartridge according to claim 5,
wherein the connecting portion is a portion of the enclosing body and is a portion extended from the sealing portion toward the supply port.

7. The assay cartridge according to claim 5,
wherein the connecting portion is a tubular body separated from the enclosing body.

8. The assay cartridge according to any one of claims 3 to 7,
wherein in a case where the enclosing body includes a plurality of sealing portions, one of the plurality of the sealing portions is a weak sealing portion having a weaker sealing force than the other sealing portions, and
the weak sealing portion is disposed at the supply port or is connected to the supply port through the connecting portion.

9. The assay cartridge according to any one of claims 1 to 8,
wherein the sealing portion is welded.

10. The assay cartridge according to claim 9,
wherein the sealing portion is heat-welded.

11. The assay cartridge according to claim 9 or 10 citing claim 8,
wherein sealing forces of the weak sealing portion and the other sealing portions are adjusted by controlling an applied thermal energy.

12. The assay cartridge according to any one of claims 9 to 11 citing claim 8,
wherein the weak sealing portion has a smaller welding area than the other sealing portions.

13. The assay cartridge according to any one of claims 8 to 12,
wherein the enclosing body is formed of one sheet rolled into a cylindrical shape,
the enclosing body includes two sealing portions that is formed along a direction intersecting a cylinder axis direction of the sheet and that is used for defining an enclosing body in which the reagent is to be enclosed, and
one of the two sealing portions is the weak sealing portion.
